(19) Europäisches Patentamt
European Patent Office
Office européen des brevets

(11) Numéro de publication: **0 037 352 B1**

(12) # FASCICULE DE BREVET EUROPEEN

(45) Date de publication du fascicule du brevet: **11.07.84**

(51) Int. Cl.³: **C 07 D 263/58**

(21) Numéro de dépôt: **81420044.0**

(22) Date de dépôt: **25.03.81**

(54) **Procédé de préparation de dérivés chlorés de la benzoxazolone.**

(30) Priorité: **27.03.80 FR 8007262**

(43) Date de publication de la demande: **07.10.81 Bulletin 81/40**

(45) Mention de la délivrance du brevet: **11.07.84 Bulletin 84/28**

(84) Etats contractants désignés: **AT BE CH DE FR GB IT LI LU NL SE**

(56) Documents cités:
**CHEMICAL ABSTRACTS, vol. 88, no. 5, 30 janvier 1978, page 503, abrégé 37784b, Columbus, Ohio, US**
**JOURNAL OF THE CHEMICAL SOCIETY, (B) 1971, Londres, GB, P.B.D. DE LA MARE et al.: "The kinetics and mechanisms of aromatic halogen substitution. Part XXIX. Chlorination of aromatic hydrocarbons in slightly aqueous (96%) dioxan", pages 90-94**
**CHEMICAL ABSTRACTS, vol. 72, no. 3, 19 janvier 1970, page 341, abrégé 12704g, Columbus, Ohio, US**

***Le dossier contient des informations techniques*** **présentées postérieurement au dépôt de la demande et ne figurant pas dans le présent fascicule.**

(73) Titulaire: **RHONE-POULENC AGROCHIMIE, 14-20, rue Pierre Baizet, F-69009 Lyon (FR)**

(72) Inventeur: **Rouy, Noel, 40, Rue Pierre Guilbert, F-91330 Yerres (FR)**
Inventeur: **Dewilde, François, 13, Avenue Hoche, F-94320 Thiais (FR)**

(74) Mandataire: **Brachotte, Charles et al, RHONE-POULENC AGROCHIMIE BP 9163-09, F-69263 Lyon Cedex 1 (FR)**

EP 0 037 352 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

**Description**

La présente invention concerne un procédé de préparation de dérivés chlorés de la benzoxazolone utilisables notamment comme intermédiaires dans la préparation d'insecticides.

Plus précisément l'invention concerne la préparation de dérivés de type chloro-6 benzoxazolone ayant pour formule

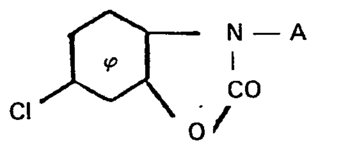

(I)

dans laquelle A représente un atome d'hydrogène ou un groupe hydroxyméthyle. Lorsque A est l'atome d'hydrogène, le dérivé est la chloro-6 benzoxazolone; lorsque A est un groupe hydroxyméthyle, le dérivé est l'hydroxyméthyl-3 chloro-6 benzoxazolone.

Un but de l'invention est de permettre l'obtention de dérivés de formule (I) avec de bons rendements à partir de benzoxazolone comme produit ou réactif de départ.

Par benzoxazolone on entend le produit de formule:

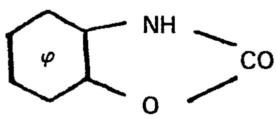

Ce produit est quelquefois appelé, notamment en langue anglaise, la benzoxazolinone.

Un autre but de l'invention est de permettre l'utilisation de benzoxazolone humide, qui est une forme de benzoxazolone obtenue de manière commode et économique sur le plan industriel.

Dans le brevet russe n° 245 111 on a déjà décrit la chloration de la benzoxazolone dans des alcanes ou alcènes chlorés tels que de tétrachloroéthane.

Il est aussi connu que la benzoxazolone se transforme en hydroxyméthyl-3 benzoxazolone par action de formaldéhyde en solution aqueuse [Zinner et al. Ber. *89* 2135 (1956)].

Toutefois ces divers procédés ne sont pas satisfaisants pour diverses raisons liées notamment à la pureté des produits obtenus ou au taux de transformation limité de la réaction.

Un but de l'invention est de fournir un procédé de préparation de dérivés de formule (I) n'ayant pas les inconvénients des procédés connus.

Un autre but de l'invention est de fournir un procédé de préparation de dérivés de formule (I) évitant autant que possible la formation de produits anormalement chlorés tels que les dérivés polychlorés sur le noyau aromatique.

Un autre but de l'invention est de faciliter la séparation et la purification des produits de formule (I) obtenus en fin de réaction.

Un autre but de l'invention est de fournir un procédé de préparation de produits de formule (I) permettant, en fin de réaction, une récupération aisée des solvents éventuellement mis en oeuvre au cours de la réaction.

Un autre but de l'invention est de fournir un moyen d'enchaîner commodément diverses opérations nécessaires pour accéder à certains intermédiaires de préparation de la phosalone.

Il a maintenant été trouvé qu'on pouvait atteindre ces buts grâce au procédé faisant l'objet de la présente invention.

Ce procédé selon l'invention est un procédé de préparation de chloro-6 benzoxazolone par réaction de chlore avec de la benzoxazolone caractérisé en ce que l'on effectue la réaction en présence d'un mélange eau + dioxanne comprenant entre 20 et 80% de dioxanne.

Le mélange eau + dioxanne utilisé dans le procédé de l'invention comprend de préférence, plus de 50% de dioxanne.

La quantité de benzoxazolone mise en oeuvre dans la réaction est généralement comprise entre 50 et 500 g/l (grammes par litre de mélange réactionnel), et de préférence entre 150 g/l et 400 g/l.

Le chlore est généralement mis en oeuvre par introduction progressive dans le milieu réactionnel où il se dissout et/ou réagit rapidement; la durée de réaction peut être très variable mais pour ces raisons économiques elle est généralement comprise entre 1 heure et 15 heures, de préférence entre 2 et 10 heures.

La température de réaction est généralement comprise entre —20°C et +90°C, de préférence entre 40° et 80°C.

La réaction est poursuivie avantageusement jusqu'à obtention d'un rendement maximal en chloro-6 benzoxazolone; l'évolution de la réaction peut être suivie par tout moyen connu en soi, par exemple la potentiométrie. La présence d'un milieu réactionnel aqueux est précisément avantageuse en ce qu'elle permet commodément de suivre l'évolution de la réaction par potentiométrie.

On peut aussi adapter la quantité de chlore mise en oeuvre de manière à obtenir ce rendement maximal en chloro-6 benzoxazolone. La quantité de chlore mise en oeuvre est avantageusement voisine de la stoechiométrie; généralement elle est comprise entre 1 et 1,1 mole/mole (c'est-à-dire mole de chlore par mole de benzoxazolone mise en oeuvre), de préférence entre 1,03 et 1,07 mole/mole.

Au cours de la réaction il se forme de l'acide chlorhydrique qui se dissout au moins partiellement dans le milieu réactionnel mais qui peut aussi se dégager à l'état gazeux.

En fin de réaction le milieu réactionnel est avantageusement neutralisé par un agent basique, par exemple un hydroxyde ou carbonate alcalin ou alcalinoterreux, de préférence de sodium ou potassium.

Le plus souvent, la présence des différents produits de réaction et de neutralisation, produit un relargage c'est-à-dire la séparation du milieu réactionnel en deux phases liquides, l'une essentiellement organique, l'autre essentiellement aqueuse.

Selon une première variante de l'invention, la chloro-6 benzoxazolone est isolée du milieu réactionnel par tout moyen connu en soi, par exemple par cristallisation.

Selon une modalité commode de réalisation de l'invention, on peut régler le rendement et la pureté de la chloro-6 benzoxazolone obtenue en modifiant les

conditions de cristallisation et notamment le rapport eau/dioxanne et la température lors de cette cristallisation et de la filtration qui suit.

Selon une autre variante de procédé de l'invention, on fait réagir la chloro-6 benzoxazolone avec du formaldéhyde pour former de l'hydroxyméthyl-3 chloro-6 benzoxazolone.

Cette variante du procédé selon l'invention est donc un procédé de préparation d'hydroxyméthyl-3 chloro-6 benzoxazolone caractérisé en ce qu'on fait réagir la chloro-6 benzoxazolone en solution organique, obtenue selon le procédé décrit ci-dessous, avec du formaldéhyde en présence d'eau et de dioxanne.

Selon une modalité spécialement avantageuse, la chloro-6 benzoxazolone est mise en oeuvre sous forme de la couche organique obtenue en fin de la réaction précédente, ce qui permet d'éviter toute cristallisation ou autre forme d'isolement de la chloro-6 benzoxazolone.

Le formaldéhyde mis en oeuvre peut être sous la forme de paraformaldéhyde ou plus simplement sous forme de solution aqueuse (formol).

La réaction est effectuée entre 20 et 100°C, de préférence entre 60 et 90°C; le formaldéhyde est utilisé avantageusement en excès par rapport à la chloro-6 benzoxazolone. Le rapport molaire

$$\frac{\text{formaldéhyde}}{\text{chloro-6 benzoxazolone}}$$

est généralement compris entre 0,8 et 2, de préférence entre 1 et 1,3.

La concentration en chloro-6 benzoxazolone mise en oeuvre dans la réaction est comprise dans les mêmes zones que celles indiquées pour la benzoxazolone dans l'étape réactionnelle précédente; il en est également de même pour la teneur relative en eau et en dioxanne.

En fin de réaction, l'hydroxyméthyl-3 chloro-6 benzoxazolone est isolée par tout moyen connu en soi, par exemple par cristallisation. Comme dans l'étape précédente on peut régler le rendement et le degré de pureté de l'hydroxyméthyl-3 chloro-6 benzoxazolone en modifiant la teneur relative eau/dioxanne et la température auxquelles on effectue les opérations de cristallisation/filtration.

L'hydroxyméthyl-3 chloro-6 benzoxazolone peut être convertie en chlorométhyl-3 chloro-6 benzoxazolone à l'aide d'agents connus de chloruration ($PCl_3$, $SOCl_2$ et autres) et la chlorométhyl-3 chloro-6 benzoxazolone peut servir elle-même à donner naissance à des produits insecticides (phosalone) selon des procédés connus.

Les exemples suivants donnés à titre non limitatif, illustrent l'invention et montrent comment elle peut être mise en oeuvre.

*Exemple 1*

Dans un réacteur de 2 litres, on charge:
- 335 g d'eau
- 457 g de dioxanne
- 270 g de benzoxazolone.

On chauffe le milieu à une température de 40°C et l'on injecte dans ce milieu, par le moyen d'une tubulure en verre, du chlore avec un débit de 10 l/h jusqu'à ce qu'une quantité de 147 g ait été absorbée par le milieu réactionnel (réaction suivie par potentiométrie).

Le milieu réactionnel est neutralisé à l'aide de 246 g d'une solution aqueuse d'hydroxyde de sodium de concentration pondérale égale à 30%. La température étant alors portée à 85°C, il se produit un relargage; la phase organique supérieure, qui contient principalement du dioxanne et de la chloro-6 benzoxazolone, est séparée par décantation. On lui ajoute 250 g d'eau, on chauffe jusqu'à 80°C ce qui rend le milieu homogène et on refroidit à 20°C ce qui provoque la cristallisation de la chloro-6 benzoxazolone. Par filtration on obtient cette dernière avec un rendement de 85,7% (par rapport à la benzoxazolone mise en oeuvre) et un degré de pureté de 99%.

*Exemple 2*

On reproduit l'exemple 1 jusqu'à l'étape du relargage. On sépare la phase organique par décantation. A cette phase organique on ajoute environ 1 $cm^3$ de solution aqueuse d'hydroxyde de sodium à 30% en sorte que le pH devient égale à 4,5; on ajoute encore 202 $cm^3$ d'une solution aqueuse de formaldéhyde de concentration pondérale égale à 30%. On chauffe à 65°C pendant une demi-heure.

En fin de réaction, on ajoute 235 g d'eau et on refroidit, ce qui provoque la cristallisation de l'hydroxyméthyl-3 chloro-6 benzoxazolone qui est ainsi obtenue avec un rendement de 83% (par rapport à la benzoxazolone initialement mise en oeuvre au début de l'exemple 1) et un degré de pureté de 99,5%.

**Revendications**

1. Procédé de préparation de chloro-6 benzoxazolone par réaction de chlore avec de la benzoxazolone caractérisé en ce que l'on effectue la réaction en présence d'un mélange eau + dioxanne comprenant entre 20 et 80% de dioxanne.

2. Procédé selon la revendication 1, caractérisé en ce que le mélange eau + dioxanne comprend plus de 50% de dioxanne.

3. Procédé selon l'une des revendications 1 à 2, caractérisé en ce que la quantité de benzoxazolone mise en oeuvre est comprise entre 50 et 500 g/l de mélange réactionnel.

4. Procédé selon la revendication 3, caractérisé en ce que la quantité de benzoxazolone mise en oeuvre est comprise entre 150 et 400 g/l.

5. Procédé selon l'une des revendications 1 à 4, caractérisé en ce que la température de réaction est comprise entre 40 et 80°C.

6. Procédé selon l'une des revendications 1 à 5, caractérisé en ce que la quantité de chlore mise en oeuvre est comprise entre 1 et 1,1 mole par mole de benzoxazolone.

7. Procédé de préparation d'hydroxyméthyl-3 chloro-6 benzoxazolone, caractérisé en ce que l'on fait réagir la chloro-6 benzoxazolone en solution organique obtenue selon l'une des revendications 1 à 6 avec du formaldéhyde en présence d'eau et de dioxanne.

8. Procédé selon la revendication 7, caractérisé en ce que la teneur respective eau/dioxanne est comprise dans les limites définies aux revendications 1 ou 2.

9. Procédé selon l'une des revendications 7 ou 8 caractérisé en ce que le formaldéhyde est en solution aqueuse.

10. Procédé selon l'une des revendications 7 à 9, caractérisé en ce que le rapport molaire formaldéhyde/chloro-6 benzoxazolone est compris entre 0,8 et 2.

11. Procédé selon la revendication 10, caractérisé en ce que le rapport molaire est compris entre 1 et 1,3.

12. Procédé selon l'une des revendications 7 à 11, caractérisé en ce que la quantité de chloro-6 benzoxazolone mise en oeuvre est comprise entre 50 et 500 g/l de mélange réactionnel et que la température réactionnelle est comprise entre 20 et 100°C.

13. Procédé selon la revendication 12, caractérisé en ce que la quantité de chloro-6 benzoxazolone mise en oeuvre est comprise entre 150 et 400 g/l et que la température réactionnelle est comprise entre 60 et 90°C.

**Claims**

1. A process for the preparation of 6-chlorobenzoxazolone by the reaction of benzoxazolone with chlorine, characterised in that the reaction is carried out in the presence of a mixture of water and dioxan containing between 20 and 80 per cent of dioxane.

2. A process according to claim 1, characterised in that the mixture of water + dioxane comprises more than 50% of dioxane.

3. A process according to one of claim 1 to 2, characterised in that the amount of benzoxazolone used is between 50 and 500 g/litre of reaction mixture.

4. A process according to claim 3, characterised in that the amount of benzoxazolone used is between 150 and 400 g/litre.

5. A process according to one of claims 1 to 4, characterised in that the reaction temperature is between 40 and 80°C.

6. A process according to one of claims 1 to 5, characterised in that the amount of chlorine added is between 1 and 1.1 mols per mol of benzoxazolone.

7. A process for the preparation of a 3-hydroxymethyl-6-chloro benzoxazolone, characterised in that 6-chlorobenzoxazolone in organic solution obtained according to one of claims 1 to 6 is reacted with formaldehyde in the presence of water and dioxane.

8. A process according to claim 7, characterised in that the respective water/dioxane ratio is within the limits defined in claims 1 or 2.

9. A process according to one of claims 7 or 8, characterised in that the formaldehyde is in aqueous solution.

10. A process according to one of claims 7 to 9, characterised in that the molar ratio of formaldehyde/6-chlorobenzoxazolone is between 0.8 and 2.

11. A process according to claim 10, characterised in that the molar ratio is between 1 and 1.3.

12. A process according to one of claims 7 to 11, characterised in that the amount of 6-chlorobenzoxazolone used is between 50 and 500 g/litre of reaction mixture and the reaction temperature is between 20 and 100°C.

13. A process according to claim 12, characterised in that the amount of 6-chlorobenzoxazolone used is between 150 and 400 g/litre and the reaction temperature is between 60 and 90°C.

**Patentansprüche**

1. Verfahren zur Herstellung von 6-Chlor-benzoxazolon durch Reaktion von Chlor mit Benzoxazolon dadurch gekennzeichnet, dass man die Reaktion in Gegenwart eines Gemisches aus Wasser und Dioxan, enthaltend 20 bis 80% Dioxan, durchführt.

2. Verfahren nach Anspruch 1, dadurch gekennzeichnet, dass das Gemisch aus Wasser und Dioxan mehr als 50% Dioxan enthält.

3. Verfahren nach einem der Ansprüche 1 bis 2, dadurch gekennzeichnet, dass die eingesetzte Menge an Benzoxazolon 50 bis 500 g/l Reaktionsgemisch ausmacht.

4. Verfahren nach Anspruch 3, dadurch gekennzeichnet, dass die eingesetzte Menge an Benzoxazolon 150 bis 400 g/l ausmacht.

5. Verfahren nach einem der Ansprüche 1 bis 4, dadurch gekennzeichnet, dass die Reaktionstemperatur 40 bis 80°C beträgt.

6. Verfahren nach einem der Ansprüche 1 bis 5, dadurch gekennzeichnet, dass die eingesetzte Menge Chlor 1 bis 1,1 Mol/Mol Benzoxazolon ausmacht.

7. Verfahren zur Herstellung von 3-Hydroxymethyl-6-chlor-benzoxazolon dadurch gekennzeichnet, dass man das gemäss einem der Ansprüche 1 bis 6 in organischer Lösung erhaltene 6-Chlor-benzoxazolon mit Formaldehyd in Gegenwart von Wasser und Dioxan umsetzt.

8. Verfahren nach Anspruch 7, dadurch gekennzeichnet, dass der jeweilige Gehalt an Wasser und Dioxan innerhalb der in den Ansprüchen 1 oder 2 definierten Grenzen liegt.

9. Verfahren nach einem der Ansprüche 7 oder 8, dadurch gekennzeichnet, dass der Formaldehyd als wässrige Lösung vorliegt.

10. Verfahren nach einem der Ansprüche 7 bis 9, dadurch gekennzeichnet, dass das Mol-Verhältnis von Formaldehyd zu 6-Chlor-benzoxazolon 0,8 bis 2 beträgt.

11. Verfahren nach Anspruch 10, dadurch gekennzeichnet, dass das Mol-Verhältnis 1 bis 1,3 beträgt.

12. Verfahren nach einem der Ansprüche 7 bis 11, dadurch gekennzeichnet, dass die eingesetzte Menge an 6-Chlor-benzoxazolon 50 bis 500 g/l Reaktionsgemisch ausmacht und die Reaktionstemperatur 20 bis 100°C beträgt.

13. Verfahren nach Anspruch 12, dadurch gekennzeichnet, dass die eingesetzte Menge 6-Chlor-benzoxazolon 150 bis 400 g/l ausmacht und die Reaktionstemperatur 60 bis 90°C beträgt.